# EUROPEAN PATENT APPLICATION

(11) **EP 2 428 122 A1**
(43) Date of publication of application: **14.03.2012**
(21) Application number: 10176117.9
(22) Date of filing: 10.09.2010
(51) Int. Cl.: A23L 1/236, A23L 1/30, C07K 14/43

(54) **A thaumatin-based improved sweetening composition and edible products made therewith**

(71) Applicant: Nestec S.A., 1800 Vevey (CH)
(72) Inventor: Galindo Cuspinera, Veronica, 3511RA, UTRECHT (NL); Godinot, Nicolas, BEIJING, 100125 (CN); Martin, Nathalie Marguerite Claire, 1068, LES MONTS DE PULLY (CH)
(74) Representative: Marquardt, Ulf

(57) **Abstract**

The present invention is directed to a composition for sweetening an edible product, comprising thaumatin, characterized in that it further comprises gymnemic acid.

## Description

### Field of the invention

The present invention concerns a sweetening composition comprising a thaumatin extract, which has improved taste properties.

### Background of the invention

In order to replace sugar, especially cane sugar (sucrose), many different molecules are used, either natural or artificial. The interest of such molecules is that the sweetening effect can be obtained in the final food composition while at the same time guaranteeing a much lower caloric intake for the consumer.

Artificial sweeteners, although widely used, show a tendency to be replaced recently by other compounds, which are extracted directly from plants. Such natural sweeteners offer similar sweetening properties, a low caloric intake to the consumer, and they are not produced by heavy chemical processes which is seen by the consumers as a clear advantage.

Amongst other natural sweeteners, Thaumatin is known for a long time for its sweetening properties. It has a long history of use in Africa, where the Katemfe fruit is grown and out of which the thaumatin is extracted. It is also used in Asia for these sweetening properties, and recently, it has gained a lot of interest in North America and Europe for it is extracted directly from a fruit, and is therefore a natural ingredient, compared to other known sweeteners, like artificial sweeteners such as aspartame or saccharine for instance.

In the following description, for the sake of simplicity, the word "thaumatin" is used to designate the different sweetening proteins that can be extracted from the Katemfe fruit (Thaumatoccocus Danielli). It is therefore meant to cover the use of one of the various thaumatin proteins (thaumatin I, II, III, a, b, or c, or a mixture thereof).

One drawback of Thaumatin though, is its relatively strong lingering sweet aftertaste. This aftertaste is structure-related and is made evident by the fact that the sweetening taste can be perceived a long time after the Thaumatin was in contact with the tongue (up to 2 hours).

In order to mask this aftertaste, different solutions have been tested and commercialised, which are directed to mixing Thaumatin with flavouring compounds and aromas that create a specific organoleptic profile. For instance, the Thaumatin can be mixed as a powder with a vanilla flavour. However, although the aromatic profile of such mixes can prove to be pleasant to taste, in practice, it does not solve the lingering taste issue.

The main objective of the present invention is therefore to provide an allnatural sweetening composition based on thaumatin, with similar or unchanged sweetening intensity, while solving the lingering sweet taste issue described above.

### Summary of the invention

The above objective is met with a composition for sweetening an edible product, comprising thaumatin, characterized in that it further comprises gymnemic acid.

It has been found that surprisingly, when a thaumatin extract is mixed in certain concentrations with gymnemic acid, the lingering sweet aftertaste usually present in Thaumatin alone is very much decreased. This property of gymnemic acid to decrease - in some conditions to stop - the lingering sweet aftertaste of thaumatin was moreover found to have a very limited effect on the sweetening intensity of thaumatin.

Therefore, when Thaumatin and gymnemic acid are mixed in correct concentrations - depending on the food composition they will be added to -, the sweet profile approaches very much, and is almost equal in some cases to the sweet profile of sucrose, in terms of intensity, in terms of duration of the sweetening taste perceived by the consumer, and in terms of sweetening taste quality. The fact that the sweetening taste is not reduced in intensity is particularly important since there is therefore no need to compensate lower sweetness intensity by adding sweetener.

In a highly desirable embodiment of the present invention, the ratio thaumatin to gymnemic acid is comprised between 6 to 8 parts of gymnemic acid for 1 part of thaumatin.

This specific ratio of course depends on the concentration of sweetener(s) in the final food product, and may be adapted to the taste of consumers. But it was found that this specific ratio range has the highest efficacy on selectively reducing or even suppressing the lingering aftertaste of Thaumatin, while not putting at stake the sweetness intensity. It was found that beyond this range, the effect of gymnemic acid is either too weak to mask the lingering aftertaste, or on the contrary, it not only blocks the aftertaste, but also blocks the sweetness intensity.

Preferably, said gymnemic acid is present in said composition as an extract of the Gymnema Sylvestre plant.

Advantageously, gymnemic acid is present in the composition according to the invention in a concentration comprised between 0.5 and 500 ppm, preferably between 2 and 250 ppm, and more preferably in the range of 100 to 200 ppm.

This concentration corresponds, having regard to the preferred ratio of concentrations of gymnemic acid and thaumatin, to the optimized concentrations of thaumatin that should be present in different food products to obtain an acceptable sweet taste in the latter.

In one possible alternative embodiment of the present invention, the composition further comprises at least one second sweetening agent selected from the list of:
(i) natural sweeteners such as Momordica Grosvenorii (Mogrosides IV or V), Stevia, Rebaudioside A, Brazzein, Glycyrrhyzic acid and its salts, Curculin, Monellin, Phylloducin, Rubusosides, Mabinlin, dulcoside A, dulcoside B, siamenoside, monatin and its salts (monatin SS, RR, RS, SR), hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, erythritol, and/or other natural polyols such as maltitol, mannitol, lactitol, sorbitol, inositol, Isomalt, xylitol, glycerol, propylene glycol, threitol, galactitol, reduced isomalto-oligosaccharides, palatinose, reduced xylo-oligosaccharides, reduced gentio-oligosaccharides, reduced maltose syrup, or reduced glucose syrup, or a mixture thereof, and/or
(ii) artificial sweeteners, such as Aspartame, Cyclamate, Sucralose, Acesulfame K, neotame, Saccharin, Neohesperidin dihydrochalcone, or mixtures thereof.

By adding at least one additional sweetener to the composition, the sweet taste profile can be fine-tuned and adapted to specific food compositions wherein said composition is used. This is due to the fact that artificial sweeteners are known to have synergistic effect among them and minor variations in the sweet taste perception may also occur, due to the interaction of thaumatin with other ingredients (aromas, fat components for instance) that are present in the said food compositions.

The composition according to the invention can further comprise at least one sweet taste improving carbohydrate, chosen from the list of, but not limited to, sucrose, fructose, glucose, maltose, lactose, mannose, galactose, ribose, rhamnose, trehalose, tagatose, high fructose corn syrup (HFCS).

The composition according to the invention can be a powder to be added directly into a food product with other ingredients. Alternatively, it can be a liquid, viscous, or at least partly jellified composition suitable for being sprayed onto and/or injected into an edible product.

The present invention is further directed to a sweetened food composition comprising at least a thaumatin as a sweetening agent, characterized in that said food composition further comprises gymnemic acid as a sweet lingering taste stopper.

It is highly preferable in the field of the present invention that the ratio gymnemic acid to thaumatin is comprised between 6 to 8 parts of gymnemic acid for 1 part of thaumatin.

Further, gymnemic acid is advantageously present in said composition as an extract of Gymnema Sylvestre.

Advantageously, said gymnemic acid is present in a concentration comprises between 0.5 and 500 ppm, preferably between 2 and 250 ppm, and more preferably in the range of 100 to 200 ppm.

In one embodiment of the invention, the food composition can further comprise at least one second sweetening agent selected from the list of:
(i) natural sweeteners such as Momordica Grosvenorii (Mogrosides IV or V), Stevia, Rebaudioside A, Brazzein, Glycyrrhyzic acid and its salts, Curculin, Monellin, Phylloducin, Rubusosides, Mabinlin, dulcoside A, dulcoside B, siamenoside, monatin and its salts (monatin SS, RR, RS, SR), hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, erythritol, and/or other natural polyols such as maltitol, mannitol, lactitol, sorbitol, inositol, Isomalt, xylitol, glycerol, propylene glycol, threitol, galactitol, reduced isomalto-oligosaccharides, palatinose, reduced xylo-oligosaccharides, reduced gentio-oligosaccharides, reduced maltose syrup, or reduced glucose syrup, or a mixture thereof, and/or
(ii) artificial sweeteners, such as Aspartame, Cyclamate, Sucralose, Acesulfame K, neotame, Saccharin, Neohesperidin dihydrochalcone, or mixtures thereof.

In another embodiment of the invention, the food composition can further comprise at least one sweet taste improving carbohydrate, chosen from the list of, but not limited to, sucrose, fructose, glucose, maltose, lactose, mannose, galactose, ribose, rhamnose, trehalose, tagatose, high fructose corn syrup (HFCS)

The food composition according to the present invention can be:
(i) a ready-to-drink beverage comprising at least coffee, tea, cocoa, cereals, or a combination thereof, or
(ii) a water-based beverage comprising, at least one natural or artificial flavor, at least one fruit extract, at least one preservative, at least one mineral salt, or a combination thereof.

It can also be a chilled dairy product comprising a fresh cheese, a dessert comprising at least one dairy component, an ice cream, a dairy culinary preparation, a sweetened condensed milk or a combination thereof.

Alternatively, it can be a confectionery product, or a bakery product.

### Brief description of the drawings

Additional features and advantages of the present invention are described in, and will be apparent from, the description of the presently preferred embodiments which are set out below with reference to the drawings in which:
**Figure 1** is a schematic diagram showing the evolution of sweetness intensity over time, between thaumatin and sucrose;
**Figure 2** is a schematic diagram illustrating the change of persistency as a function of sweetness, among different concentrations of gymnemic acid mixed with the sweetener, and wherein the reference point is the sweetener taken alone;
**Figure 3** is a schematic diagram illustrating the effect of gymnemic acid on sweetness and persistency in Thaumatin;
**Figure 4** is a schematic diagram illustrating the modifications of sweetness when very small amounts of gymnemic acid are used.

### Detailed description of the invention

One first aspect of the present invention is directed to composition for sweetening an edible product, comprising thaumatin, which further comprises gymnemic acid.

The ratio "gymnemic acid to thaumatin" is comprised between 6 to 8 parts of gymnemic acid for 1 part of thaumatin.

The gymnemic acid is present in said composition as an extract of the Gymnema sylvestre plant.

The composition according to the invention can be under powder or liquid form to be added as an ingredient into the composition of an edible food product. As an alternative, the sweetening composition according to the invention can be manufactured under a form which can be sprayed onto, or injected into, an edible food product that is already at least partially manufactured. This can be for instance a snack or a confectionery bar that contains cereals, and which requires a binding agent such as sugar syrup to bind the cereal particles and other ingredients together so that the snack bar can be manufactured by extrusion.

A second aspect of the present invention is directed to a food composition that is sweetened with Thaumatin and also comprises a predetermined and controlled amount of gymnemic acid - as an extract of Gymnema sylvestre - so as to diminish the lingering sweet aftertaste while at the same time, conserving substantially the same sweet intensity in the first few seconds of the tasting.

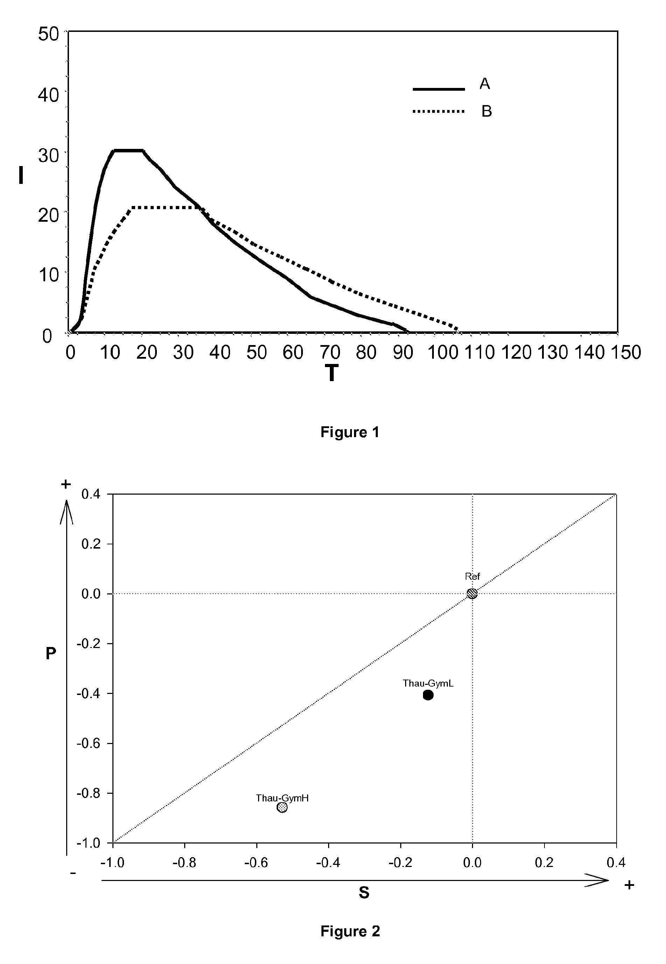

In **figure 1** is illustrated the evolution of sweetness intensity over time for sucrose (sample "A" on the drawing) and thaumatin (sample "B" on the drawing). The two samples are free of gymnemic acid. It can be seen that in absence of gymnemic acid, the persistency of the sweet taste is much higher for Thaumatin than for sucrose, even when matching the maximum sweetness of both compounds, Thaumatin still presents a much longer sweet persistency than sucrose.

Now, as illustrated in **figure 2****,** the effect of gymnemic acid on lingering sweet aftertaste is demonstrated by sensory tests that were performed by an expert panel of tasters. **Figure 2** illustrates how gymnemic acid mixed with Thaumatin, in the ratio range mentioned hereinabove (6 to 8 parts of gymnemic acid for 1 part of thaumatin), lowered the persistency so as to obtain a sweetness persistency much lower than a reference ("Ref") which corresponds for each different mix that was tested, to the same sweetener but taken alone (without gymnemic acid).

In **figure 2****,** two different samples of Thaumatin were tested, each in combination with a different concentration of gymnemic acid: one having a high concentration of 162 ppm, and the other sample having a low concentration of 122 ppm of gymnemic acid. These samples were tested versus a reference which was the pure sweetener without gymnemic acid, marked as "Ref" in **figure 2****.** On the diagram are shown two axes: the horizontal axis "S" corresponds to the perceived level of sweetness, while the vertical axis "P" corresponds to the persistency level of sweetness over time.

In the figure, the two samples that were tested are referenced to as follows: "Thau-GymnH" is a Thaumatin extract with a high concentration of gymnemic acid; "Thau-GymnL" is a Thaumatin extract with a low concentration of gymnemic acid. The reference point corresponds to the sweetener taken alone, respectively for the two Thaumatin samples.

From **figure 2****,** it is clear that gymnemic acid - whatever the concentration - has strong reducing effect on the sweetness persistency in Thaumatin, which is lowered compared to the reference (Thaumatin without gymnemic acid).

**Figure 2** also shows that gymnemic acid at a high concentration impacts negatively on the sweetness intensity, which is significantly lowered compared to the reference, and compared to the sample of thaumatin mixed with a lower concentration of gymnemic acid. The low concentration of Gymnemic acid alters slightly the sweetness, but has a stronger reduction on the lingering.

This proves that at a certain predetermined concentration that is quite low, gymnemic acid not only has an effect on the sweet lingering aftertaste reduction of thaumatin, in such a way that Thaumatin resembles the taste of sucrose in terms of sweetness persistency, but it also has little or no effect on the sweetness intensity of the Thaumatin, which is very much like that of cane sugar (sucrose). As previously said, this concentration was found to be in the range of 2 to 250 ppm and much more preferably within the range of 100 to 200 ppm. At the same time, the ratio of said gymnemic acid to sucrose should be in the range of 6 to 8 parts of gymnemic acid for 1 part of Thaumatin.

With all the testing that was done, it was found that with the mixes thus tested, the intensity is more than 80% that of sucrose, and the lingering sweet aftertaste is reduced by 28% when adding gymnemic acid, such that the sweet perception is stopped (which means that sweetness is hardly or not at all noticeable) within 100 seconds, and in some cases within 90 seconds after the product has been swallowed or spat (without rinsing the mouth).

This is achieved for minimal concentrations of gymnemic acid, which therefore do not impair the sweetness intensity, but are sufficient to ensure that gymnemic acid has a positive effect on the lingering taste reduction.

The following table gives the optimal concentrations of gymnemic acid in ppm, depending on the concentration of thaumatin in a sweetening composition or a food composition according to the invention, and for three different gymnemic acid compositions (high content of gymnemic acid and low content of the composition in gymnemic acid).

| **Thaumatin** | **Gymnemic acid Low (0.015%)** | **Gymnemic acid High (0.02%)** |
|---|---|---|
| **1 ppm** | 6.15 | 8.15 |
| **5ppm** | 30 | 40.75 |
| **10ppm** | 61.5 | 81.5 |
| **20ppm** | 120 | 163 |

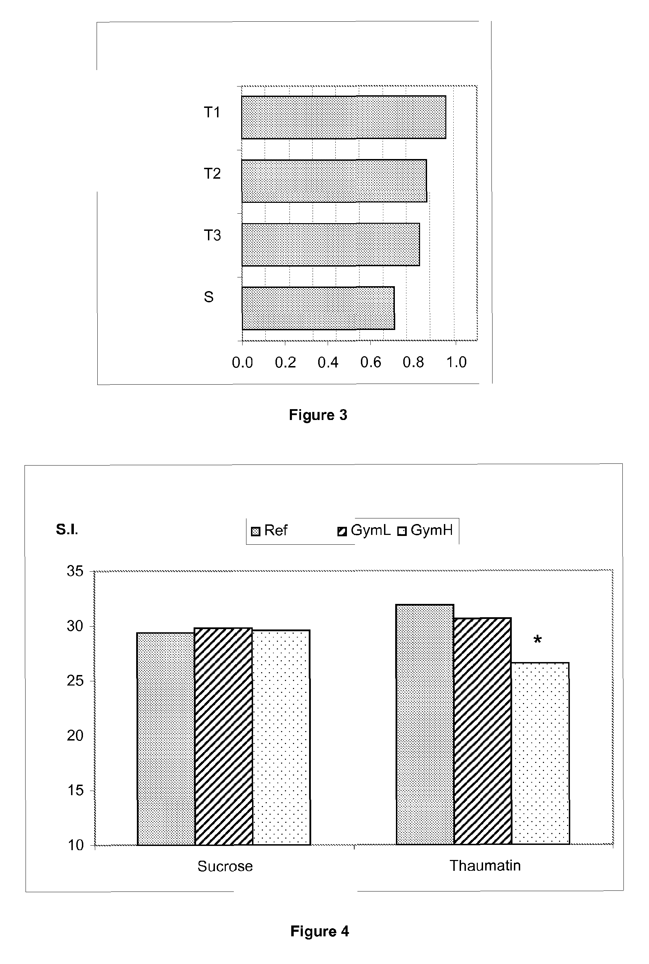

In **figure 3****,** is illustrated an assessment of the effect of gymnemic acid on the sweetness and persistency of various sweeteners samples. For the sake of the assessment, a ratio was calculated between persistency and maximum intensity, which is then represented on a scale from 0 to 1. From **figure 2****,** it is clear that for Thaumatin taken alone (sample "T1"), the persistency is very high. When gymnemic acid is added to Thaumatin at a low concentration of about 122 ppm (sample "T2"), the persistency decreases. The persistency is even more decreased when a high concentration of gymnemic acid (about 162 ppm) is added to Thaumatin (sample "T3"). In the latter case, the persistency that is measured by the expert panellists approaches that of sucrose taken alone (sample "S"). This clearly shows the effect of gymnemic acid on the sweetness persistency reduction in Thaumatin, at a range of concentrations that is ideally of about 100 to 200 ppm, according to the present invention.

In **figure 4****,** is shown that the effect of gymnemic acid at low concentrations (122 ppm) has no effect on the sweetness intensity in Thaumatin samples. However, a higher concentration of gymnemic acid (162 ppm) provokes a noticeable reduction of the sweetness, unlike what is found with the tests on sucrose where the concentration of gymnemic acid has little effect on the reduction of sweetness intensity. This proves that at the concentration of gymnemic acid that is given above (from 100 to 200 ppm), the sweetness persistency is reduced in Thaumatin, but the maximum sweetness intensity that is perceived remains almost unchanged compared to a reference (Ref) where no gymnemic acid is added.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. A composition for sweetening an edible product, comprising thaumatin, **characterized in that** it further comprises gymnemic acid.

2. A composition according to claim 1 wherein the ratio gymnemic acid to thaumatin is comprised between 6 to 8 parts of gymnemic acid for 1 part of thaumatin.

3. A composition according to any of the preceding claims, wherein said gymnemic acid is present in said composition as an extract of gymnema sylvestre; and/or wherein said thaumatin is present in said composition as a thaumatin extract.

4. A composition according to any of the preceding claims, wherein said gymnemic acid is present in a concentration comprised between 0.5 and 500 ppm, preferably between 2 and 250 ppm, and more preferably in the range of 100 to 200 ppm.

5. A composition according to any of the preceding claims, which further comprises at least one second sweetening agent selected from the list of:
(i) natural sweeteners such as Momordica Grosvenorii (Mogrosides IV or V), Stevia, Rebaudioside A, Brazzein, Glycyrrhyzic acid and its salts, Curculin, Monellin, Phylloducin, Rubusosides, Mabinlin, dulcoside A, dulcoside B, siamenoside, monatin and its salts (monatin SS, RR, RS, SR), thaumatin, hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, erythritol, and/or other natural polyols such as maltitol, mannitol, lactitol, sorbitol, inositol, Isomalt, xylitol, glycerol, propylene glycol, threitol, galactitol, reduced isomalto-oligosaccharides, palatinose, reduced xylo-oligosaccharides, reduced gentio-oligosaccharides, reduced maltose syrup, or reduced glucose syrup, or a mixture thereof, and/or
(ii) artificial sweeteners, such as Aspartame, Cyclamate, Sucralose, Acesulfame K, neotame, Saccharin, Neohesperidin dihydrochalcone, or mixtures thereof.

6. A composition according to any of the preceding claims, which further comprises at least one sweet taste improving carbohydrate, chosen from the list of, but not limited to, sucrose, fructose, glucose, maltose, lactose, mannose, galactose, ribose, rhamnose, trehalose, tagatose, high fructose corn syrup (HFCS).

7. A sweetened food composition comprising thaumatin as a sweetening agent, **characterized in that** said food composition further comprises gymnemic acid as a sweet lingering taste stopper.

8. A food composition according to claim 7, wherein the ratio gymnemic acid to thaumatin is comprised between 6 to 8 parts of gymnemic acid for 1 part of thaumatin.

9. A food composition according to any of the preceding claims 7 or 8, wherein said gymnemic acid is present in said composition as an extract of Gymnema sylvestre, and/or wherein said thaumatin is present in said composition as a thaumatin extract.

10. A food composition according to any of the preceding claims 7 to 9, wherein said gymnemic acid is present in a concentration comprised between 0.5 and 500 ppm, preferably between 2 and 250 ppm, and more preferably in the range of 100 to 200 ppm.

11. A food composition according to any of the preceding claims 7 to 10, which further comprises at least one second sweetening agent selected from the list of:
(i) natural sweeteners such as Momordica Grosvenorii (Mogrosides IV or V), Stevia, Rebaudioside A, Brazzein, Glycyrrhyzic acid and its salts, Curculin, Monellin, Phylloducin, Rubusosides, Mabinlin, dulcoside A, dulcoside B, siamenoside, monatin and its salts (monatin SS, RR, RS, SR), hernandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, erythritol, and/or other natural polyols such as maltitol, mannitol, lactitol, sorbitol, inositol, Isomalt, xylitol, glycerol, propylene glycol, threitol, galactitol, reduced isomalto-oligosaccharides, palatinose, reduced xylo-oligosaccharides, reduced gentio-oligosaccharides, reduced maltose syrup, or reduced glucose syrup, or a mixture thereof, and/or
(ii) artificial sweeteners, such as Aspartame, Cyclamate, Sucralose, Acesulfame K, neotame, Saccharin, Neohesperidin dihydrochalcone, or mixtures thereof.

12. A food composition according to any of the preceding claims 7 to 11, which further comprises at least one sweet taste improving carbohydrate, chosen from the list of, but not limited to, sucrose, fructose, glucose, maltose, lactose, mannose, galactose, ribose, rhamnose, trehalose, tagatose, high fructose corn syrup (HFCS)

13. A food composition according to any of the preceding claims 7 to 12 which is:
(i) a ready-to-drink beverage comprising at least coffee, tea, cocoa, cereals, or a combination thereof, or
(ii) a water-based beverage comprising, at least one natural or artificial flavor, at least one fruit extract, at least one preservative, at least one mineral salt, or a combination thereof.

14. A food composition according to any of the preceding claims 7 to 13 which is a chilled dairy product comprising a fresh cheese, a dessert comprising at least one dairy component, an ice cream, a dairy culinary preparation, a sweetened condensed milk or a combination thereof, a confectionery product, or a bakery product.
